# EUROPEAN PATENT SPECIFICATION

(11) **EP 0 923 878 B1**
(45) Date of publication and mention of the grant of the patent: **14.07.2004**
(21) Application number: 98310229.4
(22) Date of filing: 14.12.1998
(51) Int. Cl.: A23K 1/16, C12P 13/08

(54) **Process for making granular L-lysine feed supplement**
Verfahren zur Herstellung von granuliertem L-Lysin enthaltendem Futterzusatz
Procédé de fabrication d'un complément alimentaire pour animaux granulaire et contenant de la L-lysine

(30) Priority: 16.12.1997 US 991145; 17.06.1998 US 98948
(43) Date of publication of application: 23.06.1999
(60) Divisional application: 03028707.2
(73) Proprietor: ARCHER-DANIELS-MIDLAND COMPANY, Decatur, Illinois 62526 (US)
(72) Inventor: Stevens, Joseph Michael, Georgia 30655 (US); Binder, Thomas P., Illinois 62522 (US)
(74) Representative: Vogelsang-Wenke, Heike, Dr.

(56) References cited:
- EP-A- 0 491 638
- WO-A-95/23129
- DD-A- 139 205
- DE-C- 19 621 930
- US-A- 5 431 933

## Description

This invention relates to processes for producing a granular L-Lysine feed supplement derived from L-Lysine fermentation broth, and more particularly, to producing an L-Lysine feed supplement in which the L-Lysine content is not solely dependent on the initial L-Lysine concentration in the L-Lysine fermentation broth.

### Background Of the Invention

Lysine is an amino acid used extensively in the animal feed industry. The major form of which is L-LysineHCl (L-Lysine monohydrochloride). For many years, an L-LysineHCl solid has been produced by a process of fermentation, purification, crystallization and drying. After fermentation, the resulting broth may be rendered cell free by filtration or centrifugation. After filtration, the L-Lysine may be recovered from the fermentation broth by an ion exchange step, which produces a liquid that is substantially L-Lysine freebase. This solution may then be concentrated by evaporation.

Hydrochloric acid is usually added to the concentrated L-Lysine freebase to form L-LysineHCl. This concentrated L-LysineHCl solution is crystallized to produce a product in the form of L-LysineHCl dihydrate (L-LysineHCl:2H₂O). This crystallized solid is thereafter dried to have less than 1% moisture.

This conventional product may have shortcomings. For example, it is dusty. During the handling of the product, the dust results in a loss of valuable material and sometimes causes an incomplete formulation. Also, human working conditions are made less healthful and more difficult as a result of the dust contributed by the L-LysineHCl. In addition, the product sometimes develops lumps during storage which are difficult to break up at the time of end use. Further, the extensive use of an ion exchange step makes this process expensive.

Direct spray drying of an L-Lysine fermentation broth avoids the extensive purification steps associated with the L-Lysine hydrochloride process, in particular the use of an expensive ion-exchange step. However, consistent L-Lysine concentration in the final dry product is difficult to achieve because the L-Lysine concentration in a fermentation broth can vary considerably. Also, the dry product may be dusty and difficult to use.

Patent 5,431,933 describes a process for the production of an amino acid feed supplement which "still contains most of the solids content of the fermentation broth." The production of a fermentation broth at the industrial scale with 40% to 50% L-Lysine content is very difficult to achieve from an operational standpoint. Malfunctioning fermenters, contamination, power outages, and operator error are quite common and are likely to lead to fermentation material that is less than about 40% L-Lysine and therefore of little value. This difficulty is compounded by the impurities associated with the media components, many of which are unrefined and vary in solids content and nutrient value from lot to lot. To avoid variance in media, fermentation is constrained to specific and expensive media. These considerations may lead to an increase in operational input which is necessary to make a 40% to 50% L-Lysine product, leading to high manufacturing costs which may be prohibitive. In addition, the broth is dried to produce a powder that is difficult to handle and can have detrimental health effects on workers due to dust inhalation.

A process in which a non-dusty granular animal feed product is formed is described in patent 5,622,710. The granulation is accomplished in two steps. First, the fermentation broth is spray dried to produce particles that may include a biomass. In the second step, the particles are converted into pellets by means of costly high shear mixing equipment.

European Application Number 91460051.5 describes a method of making a granulated L-Lysine dust free, free-flowing, L-LysineHCl granular product from a liquid solution or slurry by a spray granulation process. In one embodiment of the invention, elements from a fermentation broth containing L-Lysine is ion exchanged to produce a purer L-Lysine solution. Hydrochloric acid is then added to the purer L-Lysine solution to make L-LysineHCl that is then sprayed onto an agitated drying bed of L-Lysine particulates. The particles of L-LysineHCl are then recovered once they reach a predetermined size.

International Publication Number WO/95/23129 describes the production of non-stoichiometric salt of L-Lysine in granular form. This publication teaches the production of non-stoichiometric salts of L-Lysine wherein the amount of L-Lysine content in the final product is adjustable. While the requirement for hydrochloric acid is reduced, other materials are called for such as calcium hydroxide, sulfuric acid or phosphoric acid. In addition, the fermentation broth containing the L-Lysine is extensively ion-exchanged.

DE 19621930 discloses a process for producing an animal feed additive based on a fermentation broth where the additive comprises the fermentation product, optionally mixed with preconcentrates and/or other fermentation broths.

Patent 3,089,824 describes the use of a fluidized bed for the manufacture of compressed tablets for medical use. The process comprises (1) forming a suspension of particles in air, (2) enabling the particles to be built up with granulating material, and (3) coating the resulting granules with a lubricant. In one aspect of this invention, the granulating material is atomized and sprayed into the air stream of a fluidized bed of inert particles such as sucrose. The inert particles act as nuclei for the granulation process. The resulting granules are coated with a lubricant.

This invention provides an extremely useful process as defined by the claims for making a substantially non-dusty granular L-Lysine product in which the concentration of L-Lysine in the final product is controlled by the addition of L-Lysine, which is added prior to an agglomeration step (i.e. spray granulation step). However, there are occasions where a non-granular L-Lysine feed supplement with an adjustable amount of L-Lysine purity is desirable on especially economic grounds.

An ultrafiltration step is used to provide a substantially cell free L-Lysine broth and a cell rich L-Lysine broth in the form of a permeate and a retentate respectively. If the cell rich L-Lysine broth is abandoned, there is a waste. Therefore, care should be taken either to use or to properly dispose of the cell rich L-Lysine broth. The ultrafiltration step also adds considerably to plant costs.

If the cell rich L-Lysine broth is treated as a waste by-product, it requires primary and secondary waste water treatments. If the cell rich L-Lysine broth is released as untreated sewage, there may be a deleterious impact on the environment.

For convenience of expression, the term "dryer" will hereafter be used to describe any suitable drying means such as a spray dryer, drum dryer, tunnel dryer, rotary dryer, tray dryer, and spray granulator. In addition, the term "spray granulator" will hereafter be used to describe a "Fluidized bed of particulates".

The terms "spray granulation", "spray granulation step", and "agglomeration" will hereafter be regarded as equivalent terms.

The terms "rententate" and "cell rich L-Lysine broth" will hereafter be regarded as equivalent terms.

The term "separation" will hereafter be used to describe the separating of an L-Lysine fermentation broth into two fractions: a cell rich L-Lysine broth and a substantially cell free L-Lysine broth. Any suitable separating means or combination of separating means may be used. Separation may be achieved by means of filtration (e.g. ultra and microfiltration), and mechanical methods such as centrifugation and decanting.

The term "ultrafiltration" will hereafter be used to describe the use of an ultrafilter to filter cells from an L-Lysine fermentation broth to provide a substantially cell free L-Lysine broth and a cell rich L-Lysine broth. The ultrafilter used to remove the cells, has a molecular weight cutoff between about 10,000 Dalton and 500,000 Dalton, preferably about 500,000 Dalton.

The term "neutralized L-Lysine freebase" will hereafter be used to describe a material containing L-Lysine freebase which has been neutralized using counter-ions such as Cl⁻ and SO₄²⁻. Neutralized L-Lysine freebase is obtained by reacting at least a stoichiometric amount of an acid such as hydrochloric (HCl) or sulfuric acid (H₂SO₄) with L-Lysine freebase.

The term "L-Lysine" will hereafter be used to describe at least one suitable L-Lysine used alone or combination with at least one other suitable L-Lysine. Selected from L-Lysine hydrochloride and L-Lysine sulfate.

The term "final L-Lysine feed supplement" will hereafter be used to describe a final product supplement with an L-Lysine purity within a range between about 35% and 80% L-Lysine, measured as a percent of freebase per kg. In addition, the term "final L-Lysine feed supplement" will hereafter be understood to mean a final product in which the L-Lysine in the final product is present in its neutralized form.

### Summary of the Invention

Accordingly, an object of this invention is to provide a more flexible process to produce an L-Lysine production in which the concentration of L-Lysine in the final product is controllable.

Another object of this invention is to provide an improved process for producing a substantially dust free, free flowing granular L-Lysine from fermentation broth. A more particular object is to provide a simpler and more economical method for producing granular L-Lysine. In fact, an object is to produce a substantially dust free product that does not require a high shear mixing plant. A further object is to provide an L-Lysine product that is dried and granulated in one step, a process referred to here as an "agglomeration step".

Yet another object is to produce granular L-Lysine from a fermentation broth in which the concentration of L-Lysine is adjustable by adding L-Lysine monohydrochloride (hereafter referred to as "L-Lysine hydrochloride"). A more particular object is to avoid extensive ion exchange of the fermentation broth. A further object is to provide a non-granular L-Lysine feed supplement with an adjustable amount of L-Lysine wherein the spray granulation step is replaced with alternative methods of drying such as spray drying, drum drying, rotary drying, tray drying, and tunnel drying.

### Brief Description of the Drawings

The above mentioned and other features of this invention and the manner of obtaining them will become more apparent, and the invention itself will be best understood by reference to the following description of the invention taken in conjunction with the accompanying drawings, so in which:
FIG. 1 is a flow chart, showing the principle steps in a process for producing a substantially dust free, free flowing, granular L-Lysine in the manner;
FIG. 2 is a flow chart, showing the principle steps in a process for producing an L-Lysine feed supplement in which the ultrafiltration step is optional and the water removal step is excluded;
FIG. 2A is a flow chart, showing the principle steps in a process for producing an L-Lysine feed supplement in which a variety of drying means is employed;
FIG. 3 is a flow chart, showing the principle steps in a process for producing an L-Lysine feed supplement in which there are two entry points for L-Lysine;
FIG. 3A is a flow chart, showing the principle steps in a process for producing an L-Lysine feed supplement in which a concentrated cell rich broth may be recycled for the addition of more L-Lysine ;
FIG. 4 is a flow chart, showing the principle steps in a process for producing an L-Lysine feed supplement in which an L-Lysine is added to an L-Lysine fermentation broth; and
FIG. 5 is a flow chart, showing the principle steps in a process for producing an L-Lysine feed supplement in which an L-Lysine is added to a concentrated L-Lysine broth.

### Brief Description of the Preferred Embodiments

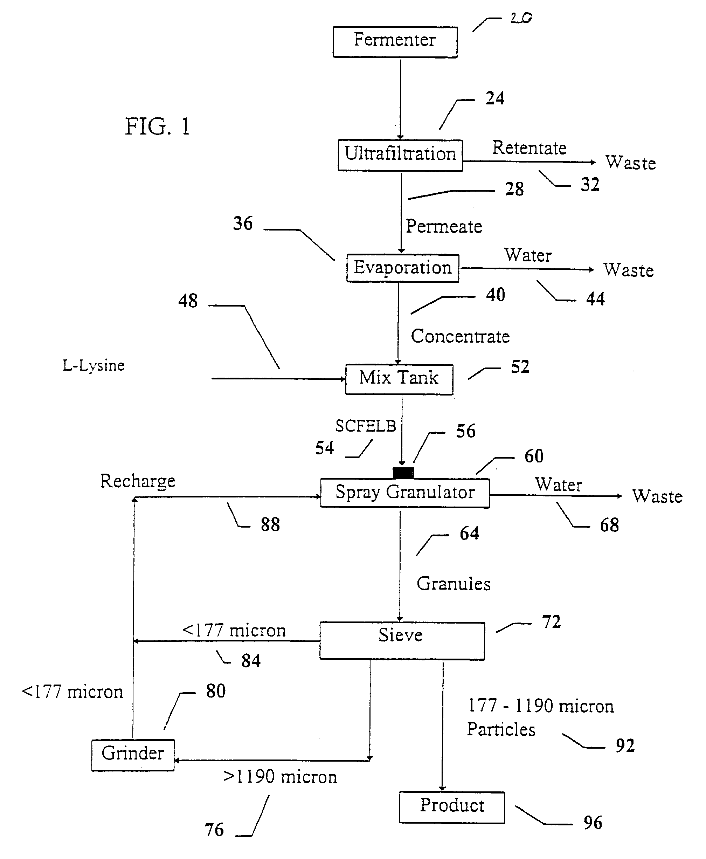

The principle steps in one embodiment is a process for producing a substantially dust free, free flowing, granular L-Lysine (FIG. 1) with an adjustable amount of L-Lysine purity in a range between about 35% and 80% L-Lysine, measured as a percent of freebase per kg. These steps comprise: (a) ultrafiltration of an L-Lysine fermentation broth to provide a substantially cell free L-Lysine permeate **28**; (b) removing water from the L-Lysine permeate of step (a) to provide a substantially cell free concentrated L-Lysine broth **40**; (c) adding L-Lysine i.e. L-Lysine hydrochloride or L-Lysine sulfate to the L-Lysine broth of step (b) to provide a substantially cell free enriched L-Lysine broth ("SCFELB") **54**; and (d) agglomerating the L-Lysine broth of step (c) to provide a feed supplement in the form of a substantially dust free, free flowing, granular L-Lysine product at **96**.

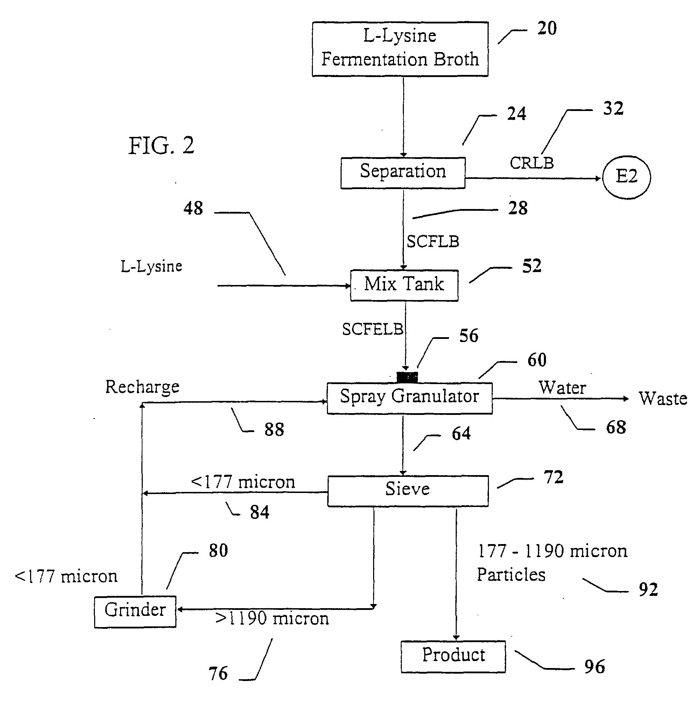

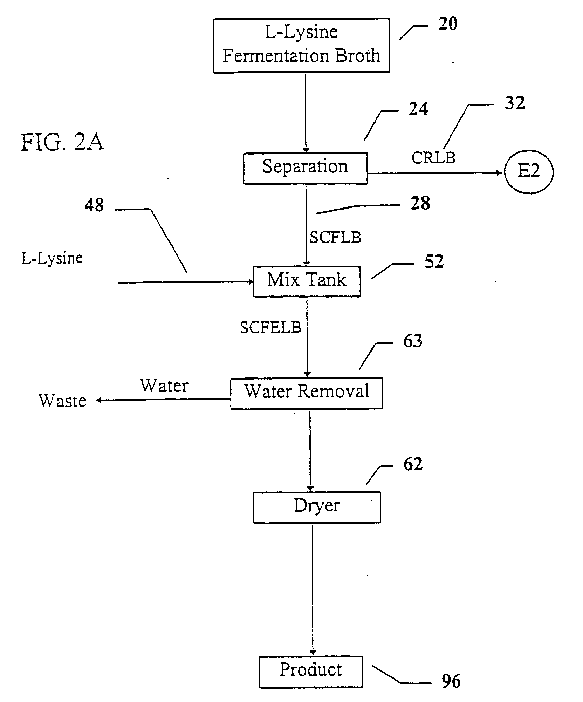

The principle steps of an inventive process (FIG. 2) described herein produces an L-Lysine feed supplement with a final L-Lysine purity in the range which is theoretically between about 35% and 80%, measured as a percent of freebase per kg, and more preferably between about 50% and 80% L-Lysine. The ultrafiltration step can be replaced with a centrifugation step and the water removal step can be excluded in a process which comprises: (a) separating, by any suitable means such as centrifugation, an L-Lysine fermentation broth into two fractions: a cell rich L-Lysine broth ("CRLB") **32** and a substantially cell free L-Lysine broth ("SCFLB") **28**; (b) adding L-Lysine, such as L-Lysine Hydrochloride , at **48** to the L-Lysine broth of step (a) in a mix tank **52** to provide a substantially cell free enriched L-Lysine broth ("SCFELB"), the added material is an amount which brings a final L-Lysine feed supplement with a L-Lysine purity to be within a range between about 35% and 80% L-Lysine, measured as a percent of freebase per kg; (c) agglomerating the L-Lysine broth of step (b) by using a spray granulator **60** to provide particles of L-Lysine; and (d) sieving the particles of step (c) to provide the final L-Lysine feed supplement **96**.

Alternatively, the substantially cell free enriched L-Lysine broth of step (ii) may be spray dried (**60** in FIG. 2A) to provide an L-Lysine feed supplement **96**. An L-Lysine feed supplement **96** may also be produced by tunnel drying, drum drying, rotary drying or tray drying the substantially cell free enriched L-Lysine broth (**62** in FIG. 2A). Excess water may be removed (**63** in FIG. 2A), and preferably removed by evaporation.

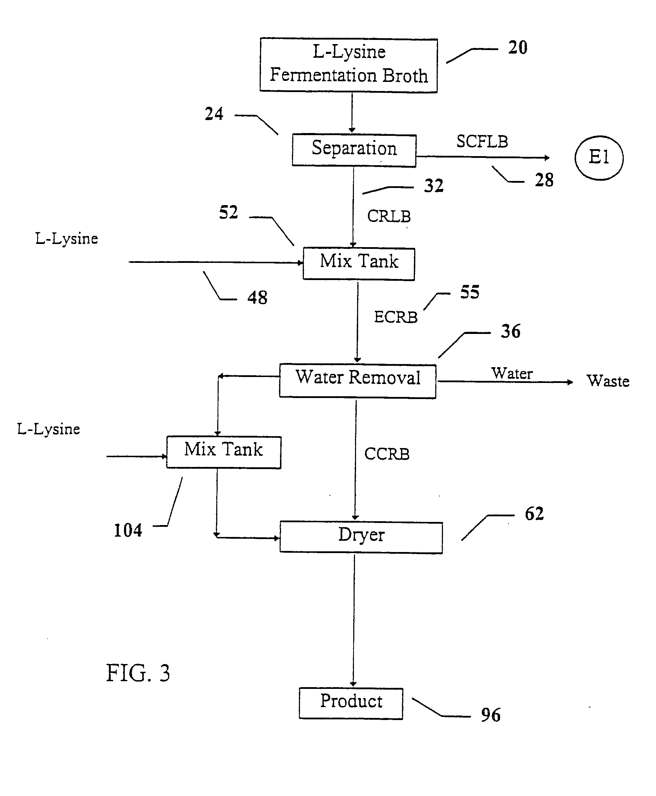

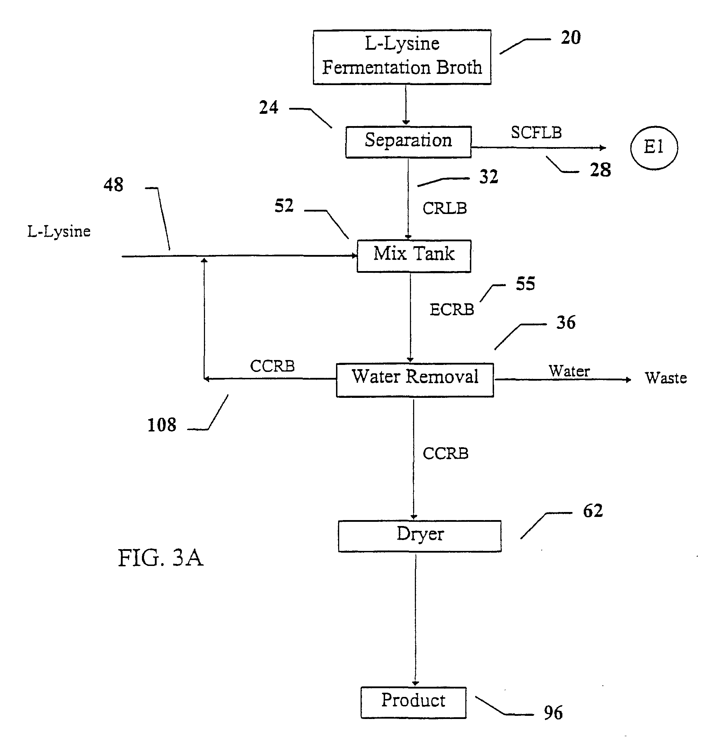

The principle steps of one aspect of the inventive process (FIG. 3) described herein produces an L-Lysine feed supplement with a final L-Lysine purity in the range theoretically between about 35% and 80%, measured as a percent of freebase per kg, and more preferably between about 50% and 80% L-Lysine. The principle steps comprises: (a) an L-Lysine fermentation broth separated into two fractions to produce a substantially cell free L-Lysine broth ("SCFLB") **28** and a cell rich L-Lysine broth ("CRLB") **32**; (b) adjusting the L-Lysine purity of the cell rich L-Lysine broth of step (a) to provide an enriched cell rich broth **52**; (c) removing water from the enriched cell rich broth of step (b) to produce a concentrated cell rich broth **36**; and (d) either drying the concentrated cell rich broth of step (c) to provide an L-Lysine feed supplement (**96**) or blending the concentrated cell rich broth of step (c) with more L-Lysine at **104** and then drying to provide an L-Lysine feed supplement at **96**. The concentrated cell rich broth may be blended with more L-Lysine on a batch or semi-batch basis as depicted in FIG. 3a.

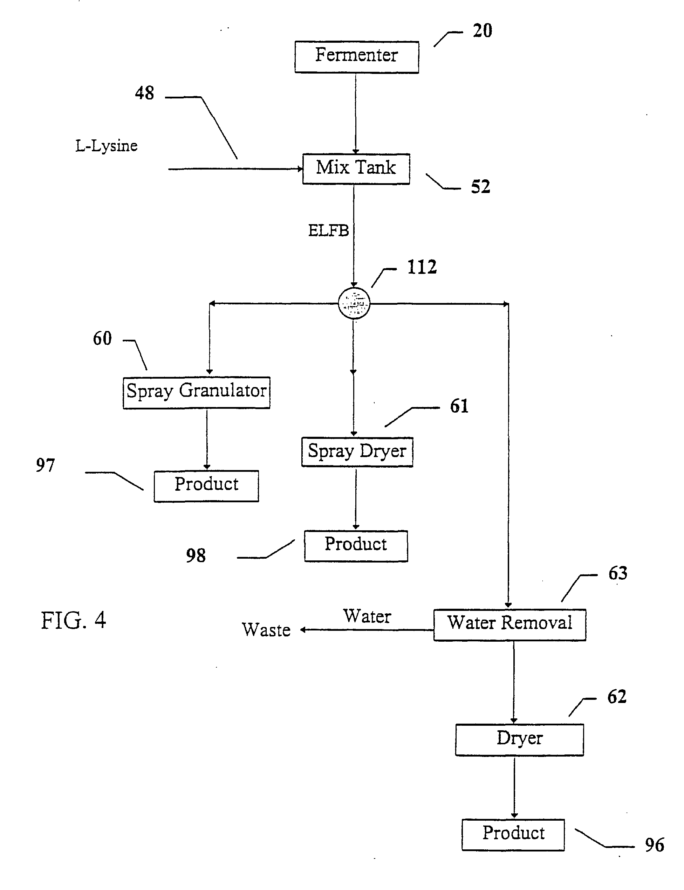

The principle steps in yet another inventive process (FIG. 4) for producing an L-Lysine feed supplement with an adjustable amount of L-Lysine purity comprises: (a) adjusting the L-Lysine purity of an L-Lysine fermentation broth to provide an enriched L-Lysine fermentation broth; and (b) converting the enriched L-Lysine fermentation broth of step (a) into an L-Lysine feed supplement by either spray granulation, spray drying, tunnel drying, tray drying, rotary drying or drum drying.

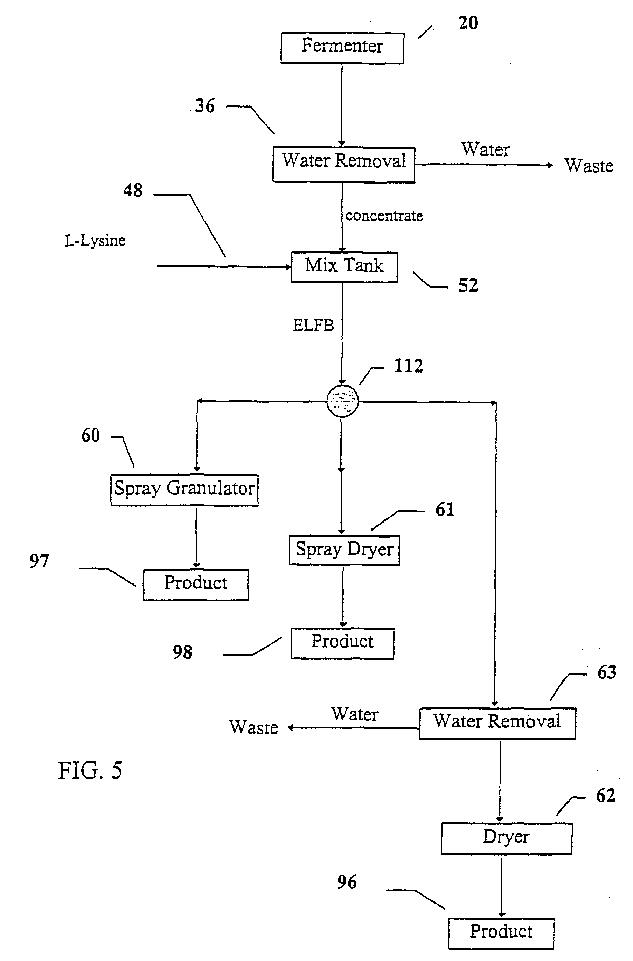

The principle steps in yet another inventive process (FIG. 5) for producing an L-Lysine feed supplement in a similar manner to that described by FIG. 4 with the optional step of removing water, preferably by evaporation, from the L-Lysine fermentation broth at **36** in order to provide a concentrated L-Lysine broth with between about 30% and 70% solids by weight. An L-Lysine is added to the concentrated L-Lysine broth at **48** to provide an enriched L-Lysine fermentation broth. The enriched L-Lysine fermentation broth may be spray granulated at **60**; spray dried at **61**; and spray dried, spray granulated, tunnel dried, tray dried, or drum dried at **62** to provide an L-Lysine feed supplement with a final L-Lysine purity in the range theoretically between about 35% and 80% L-Lysine, measured as a percent of freebase per kg, and more preferably between about 50% and 80% L-Lysine.

### Detailed Description of the Preferred Embodiments

While one aspect of this invention is the harvesting and processing of L-Lysine base from fermentation broth, the composition and nature of the fermentation medium may vary. For example, any suitable high L-Lysine producing organism taken from the genus Corynebacterium or Brevibacterium may be used to inoculate the fermentation medium. Prior to inoculation with the L-Lysine producing bacterium, the fermentation medium may have the following composition:

| Material | Amount (g/l) |
|---|---|
| Soy Hydrolysate | 20.0 |
| Ammonium Sulfate | 20.0 |
| Urea | 3.0 |
| Monopotassium Phosphate | 1.0 |
| Magnesium Sulfate Heptahydrate | 0.5 |
| Manganese Sulfate | 0.002 |
| Biotin | 0.0001 |
| Thiamine Hydrochloride | 0.0001 |
| Glucose | 30.0 |

The pH is adjusted and maintained at approximately 7.2 with ammonium hydroxide
The temperature is maintained at about 32°C
The feed is Glucose: (NH₄)₂SO₄ with the glucose concentration maintained at about 10g/l

The fermentation medium can be inoculated into the fermentation vessel by using standard microbiological practices that are known to those skilled in the microbiology art. The fermentation vessel should be equipped with a stirrer, a ventilation system, and a temperature control device to maintain the fermentation at about 30°C and preferably at 32°C. The fermentation is carried out until the L-Lysine base concentration is about 92 g/l (grams per liter) and the total dry solids is about 218 g/l. Aseptic techniques should be observed throughout the fermentation process to avoid a contamination of the fermentation broth with non-L-Lysine producing organisms.

In keeping with one aspect of this invention, which will be best understood with reference to FIG. 1, the process produces a substantially dust free, free flowing, granular L-Lysine from fermentation broth described somewhat as follows:
(i) An L-Lysine containing fermentation broth in fermenter **20** is separated into two fractions by an ultrafiltration means at **24** to remove cells in order to produce a substantially cell free L-Lysine broth (show at **28** as "Permeate" on the attached figure). The cell rich L-Lysine broth (here treated as retentate waste) is drained off at **32**. The ultrafilter used to remove the cells, has a molecular weight cut-off between 10,000 Dalton and 500,000 Dalton, preferably about 500,000 Dalton.
(ii) The substantially cell free L-Lysine broth is evaporated to remove water at **36** to produce a substantially cell free concentrated L-Lysine broth **28**. Preferably, the substantially cell free concentrated L-Lysine broth (shown as "Concentrate" at **40**) has between about 30% and 70% solids by weight. Waste water is drained away at **44**. Evaporation is carried out in the approximate range between 60°C (140°F) and 101°C (214°F), preferably between 62,8°C (145°F) and 68,3°C (155°F), and pressure between 0,2 bar (2.9 psia) and 0,76 bar (11 psia) (vacuum), preferably 0,2 bar (2.9 psia) to 0,27 bar (4 psia).
(iii) The L-Lysine purity of the substantially cell free concentrated L-Lysine broth is adjusted in a mix tank **52**. The adjustment is made by adding L-Lysine at **48** to a mix tank **52** to provide a substantially cell free enriched L-Lysine broth SCFELB at **54**. The L-Lysine is added in an amount which brings a final L-Lysine feed supplement with an L-Lysine purity to be in a range theoretically between about 35% and 80% L-Lysine, measured as a percent of freebase per kg of the final L-Lysine feed supplement, and more preferably between about 50% and 80% L-Lysine.
(iv) The substantially cell free enriched L-Lysine broth is atomized by a nozzle **56** to provide an atomized spray of substantially cell free enriched L-Lysine broth to make a percolating bed of L-Lysine particulates in a spray granulator **60**. The L-Lysine particulates have a particle size of less than about 177 micron (i.e. particles that can pass through 80 mesh) and preferably in the size range of about 100 micron and 177 micron. The bed of the spray granulator is preferably a fluidized bed of L-Lysine particulates and is operated at a temperature between about 30°C and 100°C.
(v) The position of the nozzle **56** is adjusted until it is just above the fluidized bed of L-Lysine particulates.
(vi) Substantially cell free enriched L-Lysine broth is sprayed onto the fluidized bed of L-Lysine particulates to initiate the agglomeration process.
(vii) The agglomeration process is allowed to continue to produce the substantially dust free, free flowing, granular L-Lysine product in the size range between approximately 177 micron and 1190 micron, and preferably in the size range of between about 177 micron to 420 micron.
(viii) The product is removed from the spray granulator at **64**, with waste water flowing away at **68** in the form of water vapor in the dryer exhaust.
(ix) The product is then screened and sorted for size at sieve **72** (preferably 80 mesh).
(x) Granules at **76** that are too large (e.g. in the size range of greater than about 1190 micron) are ground in a grinder at **80** to a smaller particle size (e.g. in the size range of less than about 177 micron) and combined with material that is too small **84** (e.g. in the size range of less than about 177 micron) to produce recycled L-Lysine particulates (shown at **88** as "Recharge" on FIG. 1) and returned to the spray granulator **60** as a starting material which act as seeds for the agglomeration process.
(xi) The substantially dust free, free flowing, granular L-Lysine product in the size range of about 177 micron to 1190 micron (shown at **92** as "177-1190 micron Particles") pass through the sieving process and are acceptable as the end product at **96**. However, the preferred range is from about 177 micron to 420 micron that packs better and reduces cost for shipment.

The preferred L-Lysine concentration in the starting feed stream of L-Lysine fermentation broth is about 90g/l L-Lysine, measured as a percent of freebase per kg. However, the L-Lysine concentration can vary from one fermentation run to the next. Hence, the use of a fermentation broth containing about 90 g/l L-Lysine means that other suitable concentrations of L-Lysine in the fermentation broth are acceptable. However, the L-Lysine concentration in the fermentation broth should not be below about 30 g/l. As described in step (iii) above, the final desired concentration of L-Lysine may be achieved by adding L-Lysine .

Although ultrafiltration is the preferred method for obtaining the substantially cell free L-Lysine broth, this does not mean other methods can not be used. The cells could also be removed by mechanical separation techniques, such as centrifugation. Other suitable methods include microfiltration and decanting.

The invention envisages the removal of cells from the L-Lysine containing fermentation broth by various other processes. For example, the fermentation broth **20** could be split equally and about 50% centrifuged and the remaining 50% ultrafiltered with the outputs from both cell removal processes combined to produce a substantially cell free L-Lysine broth. This flexibility will enhance the practice of the invention in an industrial setting.

Experience has shown that there is a relationship between the orifice size of the nozzle **56**, flow rate, and gauge pressure. While the preferred nozzle size is 0,15621 cm (0.0615"), various other nozzles can also be used to supply the spray. In particular, nozzle designs supplied by Spraying Systems Co., P0 Box 7900, Wheaton, IL 60189-7900, USA (tel: 630-665-5000) work well to produce a fine spray. The spray granulator can be purchased from Glatt Air Techniques, 20 Spear Road, Ramsey, NJ 07446-1288, USA (tel: 201-825-8700).

Experience also suggests that manufacturing L-Lysine granules on a commercial scale will require several nozzles to atomize and spray enriched L-Lysine broth onto a proportionally larger bed of percolating particles of L-Lysine.

The percolating bed of particles should comprise L-Lysine particles of sufficiently small size to function as seeds for the agglomeration process. It is preferable that the L-Lysine particulates are less than about 177 micron in size and preferably between about 100 micron and 177 micron.

In the agglomeration process, the seed particles simultaneously grow in size and are dried as they are sprayed with the enriched L-Lysine permeate. The agglomeration process is aided by binders that are inherently present in the enriched L-Lysine broth, namely: L-Lysine fermentation broth, L-Lysine hydrochloride, L-Lysine sulfate and water. A binder is defined as a substance that provides the sticky component to enable the seeds in the agglomeration process to build up in size.

The source of the L-Lysine particulates used to produce and seed the fluidized bed of L-Lysine in the spray granulator is not critical although the preferred source is either obtained from atomizing the substantially cell free enriched L-Lysine broth as described in step (iv) above or from recycled L-Lysine particulates as described in step (x) above, and shown at **88** in FIG. 1.

Alternatively, the fluidized bed of L-Lysine particulates could be produced by atomizing or spray drying any of the following: L-Lysine containing fermentation broth from step (i), substantially cell free L-Lysine permeate from step (i), substantially cell free L-Lysine broth produced by centrifuging L-Lysine containing fermentation broth from step (i), substantially cell free concentrated L-Lysine broth from step (ii) or any combination of these. In addition, purified L-Lysine hydrochloride and L-Lysine freebase could be used as a source for L-Lysine particulates in order to make the fluidized bed of L-Lysine particulates and to act as seeds for the agglomeration process.

While the exact source of the L-Lysine particulates for making the fluidized bed of L-Lysine particulates and the seeds for the agglomeration process is not too critical, it is preferable that the L-Lysine particulates are less than about 177 micron in size and preferably between about 100 micron and 177 micron.

In summary, suitable sources for making the L-Lysine particulates include: substantially cell free L-Lysine broth obtained from centrifuging L-Lysine containing fermentation broth from step (i), substantially cell free L-Lysine permeate from step (i), substantially cell free concentrated L-Lysine broth from step (ii), and substantially cell free enriched L-Lysine broth from step (iii). Each of these sources, or any combination of these sources, may be atomized by the nozzle **56** in the spray granulator **60** in order to generate the L-Lysine particulates. Alternatively, L-Lysine particulates may be made by separately spray drying, and possibly storing for later use, any of the following: substantially cell free L-Lysine broth obtained from centrifuging L-Lysine containing fermentation broth from step (i), substantially cell free L-Lysine permeate from step (i), substantially cell free concentrated L-Lysine broth from step (ii), and substantially cell free enriched L-Lysine broth from step (iii). Prior to use as L-Lysine particulates, the spray dried products may be sieved to remove lumps and to obtain particles in the size range of less than about 177 micron (preferably between about 100 micron and 177 micron).

For example the substantially cell free concentrated L-Lysine broth from step (ii) may be used to produce the bed of percolating seed particles at **60**. The substantially cell free concentrated L-Lysine broth may be atomized by the nozzle **56** in the spray granulator **60** in order to generate the percolating bed of seed particles. Alternatively, the substantially cell free concentrated L-Lysine broth may be separately spray dried. Prior to use as L-Lysine particulates, the spray dried concentrated L-Lysine broth may be sieved to remove lumps and to obtain particles in the size range of less than about 177 micron (preferably between about 100 micron and 177 micron).

Another example of a suitable source of the L-Lysine particulates would be dry purified L-Lysine hydrochloride powder that may be obtained by known state of the art processes. This dry powder may be used as a source for L-Lysine particulates or the dry powder may be sieved to remove lumps and sorted for particles less than about 177 micron (preferably in the size range between about 100 micron and 177 micron).

Experience has shown that once the agglomeration process becomes self-sustaining, the particles for the fluidized bed **60** comes from recycling particles at **88** into the into the fluidized spray granulator bed **60** by using particles at **84** which are too small or the granules which are too large and have been ground at **80** to a smaller size. Experience has also shown that the agglomeration process may operate on either a batch or semi-continuous basis. The batch process is preferred.

While the preferred L-Lysine concentration in the starting feed stream, measured as a percent of freebase per kg, in the fermentation broth is about 90g/l L-Lysine, those skilled in the art will understand that L-Lysine concentration can vary from one fermentation run to the next. Hence, the use of a fermentation broth containing about 90 g/l L-Lysine should not be taken to mean that other suitable concentrations of L-Lysine in the fermentation broth are precluded. The final desired concentration of L-Lysine may be achieved by adding L-Lysine , such as L-LysineHCl , at **48**, and as described in step (iii) above. However, the L-Lysine concentration in the fermentation broth should not be below about 30 g/l.

A second embodiment of this invention for producing an L-Lysine feed supplement is shown in FIG. 2.
(i) An L-Lysine containing fermentation broth in a fermenter at **20** is separated into two fractions at **24** to produce a substantially cell free L-Lysine broth ("SCFLB") **28** and a cell rich L-Lysine broth ("CRLB") **32**. The cell rich L-Lysine broth (shown as E2 in FIG. 2) may be processed as described in the third embodiment of FIG. 3. Any suitable means, such as ultrafiltration or centrifugation, may be used at **24** to separate the amino acid fermentation broth.
(ii) The L-Lysine purity of the substantially cell free L-Lysine broth is adjusted by adding an effective amount of L-Lysine at **48** (FIG. 2) to the substantially cell free L-Lysine broth in a mix tank at **52** in order to provide a substantially cell free enriched L-Lysine broth ("SCFELB"). The amount of L-Lysine added at **48** depends on the concentration of L-Lysine in the substantially cell free L-Lysine broth, measured as a percent of freebase per kg. However, the amount of L-Lysine should be sufficient to ensure that the final concentration of L-Lysine in the final product is in the range between about 35% and 80% L-Lysine, measured as a percent of freebase per kg of the final L-Lysine feed supplement.
(iii) The substantially cell free enriched L-Lysine broth is optionally atomized by a nozzle **56** to provide an atomized spray of substantially cell free enriched L-Lysine broth to make a percolating bed of L-Lysine particulates in a spray granulator **60**. The L-Lysine particulates have a particle size of less than about 177 micron (i.e. particles that can pass through 80 mesh) and preferably in the size range of about 100 micron and 177 micron. The bed of the spray granulator is preferably a fluidized bed of L-Lysine particulates and is operated at a temperature between about 30°C and 100°C.
   Alternatively, the substantially cell free enriched L-Lysine broth of step (ii) in FIG. 2 may be spray dried to provide an L-Lysine feed supplement. An L-Lysine feed supplement may also be produced by tunnel drying, drum drying, rotary drying or tray drying the substantially cell free enriched L-Lysine broth (**62** in FIG. 2A). Excess water is removed (**63** in FIG. 2A), preferably by evaporation.
(iv) The position of the nozzle **56** (FIG. 2) is adjusted until it is just above the fluidized bed of L-Lysine particulates of the spray granulator.
(v) Substantially cell free enriched L-Lysine broth is sprayed onto the fluidized bed of L-Lysine particulates of the spray granulator to initiate the agglomeration process.
(vi) The agglomeration process is allowed to continue to produce the substantially dust free, free flowing, granular L-Lysine product in the size range between approximately 177 micron and 1190 micron, and preferably in the size range of between about 177 micron to 420 micron.
(vii) The product is removed from the spray granulator at **64**, with waste water flowing away at **68** in the form of water vapor in the spray granulator exhaust.
(viii) The product is then screened and sorted for size at sieve **72** (preferably 80 mesh).
(ix) Granules at **76** that are too large (e.g. in the size range of greater than about 1190 micron) are ground in a grinder at **80** to a smaller particle size (e.g. in the size range of less than about 177 micron) and combined with material that is too small **84** (e.g. in the size range of less than about 177 micron) to produce recycled L-Lysine particulates at **88** (FIG. 2) and returned to the spray granulator **60** as starting material to act as seeds for the agglomeration process.
(x) The substantially dust free, free flowing, "177-1190 micron Particles", granular L-Lysine product with an L-Lysine purity in the range between about 35% and 80% L-Lysine, measured as a percent of freebase per kg, and a size range of about 177 micron to 1190 micron at 92 pass through the sieving process and are acceptable as the end product at **96**. However, from the viewpoint of bulk density, the preferred product size is in the range between approximately 177 micron and 420 micron.

A third embodiment (FIG. 3) of this invention produces an L-Lysine feed supplement.
(i) An L-Lysine fermentation broth in fermenter **20** is separated into two fractions at **24** to produce a substantially cell free L-Lysine broth ("SCFLB") **28** and a cell rich L-Lysine broth ("CRLB") **32**. The substantially cell free L-Lysine broth (shown as El) may be processed as described in the second embodiment of FIG. 2. Any suitable means to separate the L-Lysine fermentation broth may be used such as ultrafiltration or centrifugation.
   Prior to separating the L-Lysine fermentation broth, L-Lysine may be optionally added directly to the L-Lysine fermentation. The agitation provided by a suitable stirred tank reactor ("STR") fermenter vessel would provide the necessary degree of mixing to ensure a uniform concentration of L-Lysine in the L-Lysine fermentation broth.
(ii) The L-Lysine purity of the cell rich L-Lysine broth is adjusted by adding an effective amount of L-Lysine to the cell rich L-Lysine broth in a mix tank at **52** to provide an enriched cell rich broth ("ECRB") **55**. The amount of L-Lysine added at **48** depends on the concentration of L-Lysine in the cell rich L-Lysine broth, measured as a percent of freebase per kg. However, the amount should be sufficient to ensure that the final concentration of L-Lysine in the final product is in the range between about 35% and 80% L-Lysine, measured as a percent of freebase per kg.
(iii) Water is removed from the enriched cell rich broth by evaporation at **36** to produce a concentrated cell rich broth ("CCRB"). Preferably, the concentrated cell rich broth has between about 20% and 70% solids by weight.
(iv) The concentrated cell rich broth is dried at **62** to provide an L-Lysine feed supplement **96** with an L-Lysine purity in the range between about 35% and 80% L-Lysine, measured as a percent of freebase per kg.

Alternatively, the concentrated cell rich broth is blended with more L-Lysine in a second mix tank at **104** and then dried at **62**. If this embodiment is practiced on a batch or semi-batch basis, it would be more desirable to use just one mix tank **52** simply by recycling the concentrated cell rich broth back at **108** to mix tank **52** as depicted in FIG. 3a.

A fourth embodiment of this invention (FIG. 4) includes a process of producing an Lysine feed supplement with an L-Lysine purity in the range between about 35% and 80% L-Lysine, measured as a percent of freebase per kg.
(i) The L-Lysine purity of an L-Lysine fermentation broth in fermenter **20** is adjusted by adding an effective amount of L-Lysine at **48** to a mix tank at **52** in order to provide an enriched L-Lysine fermentation broth ("ELFB"). The amount of L-Lysine added at **48** depends on the concentration of L-Lysine in the L-Lysine fermentation broth, measured as a percent of freebase per kg. However, the amount should be sufficient to ensure that the final concentration of L-Lysine in the final product is in the range between about 35% and 80% L-Lysine, measured as a percent of freebase per kg.
(ii) Depending on the position of flow valve **112**, the enriched L-Lysine fermentation broth is either converted into a granular L-Lysine feed supplement by means of a spray granulator **60** (i.e. agglomerated) or converted into an L-Lysine feed supplement by means of a spray dryer **61**. Water is removed at **63**, preferably by evaporation.

A fifth embodiment of this invention (FIG. 5) includes a process of producing an L-Lysine feed supplement which is essentially the same as that described in the fourth embodiment with the optional step of removing water, preferably by evaporation, from the L-Lysine fermentation broth at 36 in order to provide a concentrated L-Lysine broth with between about 30% and 70% solids by weight. L-Lysine is added to the concentrated L-Lysine broth at **48** to provide an enriched L-Lysine fermentation broth. The enriched L-Lysine fermentation broth may be spray granulated at **60**; spray dried at **61**; and spray dried, spray granulated, tunnel dried, tray dried, or drum dried at **62** to provide an L-Lysine feed supplement with an L-Lysine purity in the range between about 35% and 80% L-Lysine, measured as a percent of freebase per kg.

The following examples represent specific but non-limiting embodiments of the present invention:

### EXAMPLE 1 - Comparative Example

400 liters of fermentation broth with a L-Lysine concentration of 92 g/l (grams per liter) L-Lysine base and 218 g/l total dry solids were harvested from a L-Lysine fermentation run. This material was ultrafiltered and evaporated to a concentration of 235 g/l in the form of L-Lysine sulfate (measured as free base) and 493 g/l dry solids.

5150 ml (milliliters) of this concentrate was dried on a Glatt WSG 5 spray granulator. The inlet temperature of the Glatt unit was maintained between 93°C and 124°C, preferably above 120°C. The outlet temperature was maintained between 40°C and 80°C, preferably between 60°C and 65°C. The bed temperature was maintained between 70°C and 92°C, preferably between 71°C and 74°C. The air flow was maintained between 433 m (1,300 feet) and 1333 m (4,000 feet) per minute, preferably between 433 m (1,300 feet) and 500 m (1,500 feet) per minute. The nozzle atomization air was between 3,45 to 4,83 bar (50 to 70 pound per square inch) gauge. Approximately 2,500 ml of the concentrate was sprayed into the dryer with the nozzle in the highest setting in order to form a bed of material on which to agglomerate. The nozzle was lowered to a position just above the percolating material in the bed and agglomeration was accomplished with the remaining 2,650 ml of concentrate. This yielded a granulated product having the composition indicated in Table 1.

**TABLE 1**

| Sample | +16 mesh >1190 micron | +40 mesh 420 to 1190 micron | +80 mesh 177to420 micron | -80 mesh <177micron | % Purity* |
|---|---|---|---|---|---|
| Broth | 16.1% | 58.3% | 25.5% | 0% | 46.5% |

| | | | | | |
|---|---|---|---|---|---|
| *purity measured as percent L-Lysine freebase per kg | | | | | |

### EXAMPLE 2

Lysine fermentation broth, ultrafiltered and concentrated as described above in Example 1, was mixed 4 to 1 (lysine basis) with purified L-Lysine sulfate (produced as a free base and pH adjusted to 6 with sulfuric acid yielding L-Lysine sulfate). The mixture was spray granulated as described in Example 1. The process was repeated with a 3 to 2 mixture, 2 to 3 mixture, 1 to 4 mixture, and with straight L-Lysine sulfate. The granulated products had the compositions as indicated in Table 2.

**TABLE 2**

| Sample | +16 mesh >1190 micron | +40 mesh 420 to 1190 micron | +80 mesh 177 to 420 micron | -80 mesh <177 micron | % Purity* |
|---|---|---|---|---|---|
| 4:1 | 11.6% | 50.9% | 26.1% | 11.4% | 49.0% |
| 3:2 | 28.1% | 17.1% | 49.1% | 5.7% | 52.2% |
| 2:3 | 0.9% | 40.3% | 52.5% | 6.3% | 57.4% |
| 1:4 | 6.1% | 35.0% | 41.8% | 17.1% | 62.5% |
| L-Lysine sulfate | 47.8% | 27.8% | 22.0% | 2.6% | 68.5% |

| | | | | | |
|---|---|---|---|---|---|
| * purity measured as percent L-Lysine freebase per kg | | | | | |

### EXAMPLE 3

Lysine fermentation broth, ultrafiltered and concentrated as described above in Example 1, was mixed 4 to 1 (lysine basis) with pure L-Lysine hydrochloride. The mixture was spray granulated as outlined in Example 1 above. The process was repeated with a 3 to 2 mixture, 2 to 3 mixture, 4 to 1 mixture, and with straight L-Lysine hydrochloride. The granulated products had the compositions as indicated in Table 3.

**TABLE 3**

| Sample | +16 mesh >1190 micron | +40 mesh 420 to 1190 micron | +80 mesh 177 to 420 micron | -80 mesh <177micron | % Purity* |
|---|---|---|---|---|---|
| 4:1 | 7.4% | 33.0% | 59.6% | 0% | 49.40% |
| 3:2 | 7.6% | 32.9% | 44.2% | 15.2% | 51.50% |
| 2:3 | 4.8% | 48.4% | 46.8% | 0% | 57.00% |
| 1:4 | 5.1% | 45.3% | 49.40% | 0% | 66.60% |
| L-Lysine HCl | 17.2% | 44.5% | 29% | 9.3% | 76.80% |

| | | | | | |
|---|---|---|---|---|---|
| * purity measured as percent L-Lysine freebase per kg | | | | | |

It may be seen that mixing the concentrated and ultrafiltered L-Lysine fermentation broth of Example 1 with L-Lysine sulfate or L-Lysine hydrochloride, as described in Examples 2 and 3 respectively, produces a granular product with increased L-Lysine content. Also, one preferred embodiment of the described invention enables the L-Lysine content in L-Lysine fermentation broth to be easily adjusted prior to the agglomeration step. Thus, natural variations in L-Lysine concentration, which often occur from one L-Lysine fermentation to the next L-Lysine fermentation, do not require the extensive ion exchange to obtain a final product of the necessary purity for use (e.g. as a feed additive). The preferred level of purity in the final granular L-Lysine product is in the range between about 35% and 80% L-Lysine, measured as a percent freebase per kg.

## Claims

1. A process for producing an L-lysine feed supplement comprising:
(a) determining the concentration of L-lysine, measured as a percent of freebase per kg dry matter, in an L-lysine containing fermentation broth;
(b) adding pure L-lysine which is selected from L-lysine hydro-chloride and L-lysine sulfate, to said L-lysine containing fermentation broth, said added L-lysine being an amount which brings a final L-lysine feed supplement with an L-lysine purity into a range between 35% and 80% L-lysine, measured as a percent of freebase per kg of the final L-lysine feed supplement; and
(c) substanfially drying the enriched L-lysine broth to provide the final L-lysine feed supplement.

2. The process of claim 1 wherein the L-lysine is added to a concentrated L-lysine containing fermentation broth.

3. The process of clalm 1 wherein the substantially drying of step (c) provides the final L-lysine feeds supplement with an L-lysine purity in a range between 35% and 80% L-lysine, measured as a percent of freebase per kg.

4. The processaccording to claim 2 wherein the concentrated fermentation broth is produced by evaporating an L-lysine containing fermentation broth to remove water.

5. The process according to claim 4 wherein the evaporation is carried out between 60 °C (140° F) and 101 °C (214° F).

6. The process according to claim 4 wherein the evaporation is carried out in a temperature range of 60 °C (140° F) and 101 °C (214° F) and in a vacuum of between 0.2 bar (2.9 psia) and 0.76 bar (11 psia) to provide a concentrated L-lysine broth which is between 30% and 70% solids by weight.

7. The process of any one of claims 1 to 6 wherein the substantial drying of step (c) is selected from drum drying, spray drying, rotary drying, tray drying, tunnel drying, and spray granulation.

8. The process according to claim 7 wherein the substantial drying of step (c) is spray granulation.

9. The process according to claim 7 wherein the spray granulation comprises the steps of spraying and atomizing the enriched L-lysine containing fermentation broth onto a fluidized bed of L-lysine particulates which have been formed from spray dried enriched L-lysine containing fermentation broth.

10. The process according to claim 9 wherein the L-lysine particulates are in a size range of between about 100 micron and 177 micron.

## Patentansprüche

1. Ein Verfahren zur Herstellung eines L-Lysin-Futterergänzungsmittels, umfassend:
(a) die Bestimmung der Konzentration von L-Lysin, gemessen als Prozentanteil der freien Base pro kg Trockenmasse in einer L-Lysin-haltigen Fermentationsbrühe;
(b) das Hinzufügen eines reinen L-Lysins, das aus L-Lysinhydrochlorid und L-Lysinsulfat ausgewählt ist, zu besagter L-Lysin-haltiger Fermentationsbrühe, wobei besagtes hinzugefügtes L-Lysin in einer Menge vorliegt, die ein fertiges L-Lysin-Futterergänzungsmittel mit einer L-Lysinreinheit in einem Bereich zwischen 35 % bis 80 % L-Lysin ergibt, gemessen als Prozentanteil der freien Base pro kg des fertigen L-Lysin-Futterergänzungsmittels; und
(c) das wesentliche Trocknen der angereicherten L-Lysinbrühe, um das fertige L-Lysin-Futterergänzungsmittel zur Verfügung zu stellen.

2. Das Verfahren von Anspruch 1, worin das L-Lysin zu einer konzentrierten L-Lysin-haltigen Fermentationsbrühe hinzugefügt wird.

3. Das Verfahren von Anspruch 1, worin das wesentliche Trocknen von Schritt (c) das endgültige L-Lysin-Futterergänzungsmittel mit einer L-Lysinreinheit im Bereich zwischen 35 % und 80 % L-Lysin, gemessen als Prozentanteil der freien Base pro kg, zur Verfügung stellt.

4. Das Verfahren gemäß Anspruch 2, worin die konzentrierte Fermentationsbrühe durch das Verdampfen einer L-Lysin-haltigen Fermentationsbrühe zum Entfernen von Wasser hergestellt wird.

5. Das Verfahren gemäß Anspruch 4, worin die Verdampfung zwischen 60 °C (140 °F) und 101 °C (214 °F) durchgeführt wird.

6. Das Verfahren gemäß Anspruch 4, worin die Verdampfung in einem Temperaturbereich von 60 °C (140 °F) und 101 °C (214 °F) und in einem Vakuum zwischen 0,2 bar (2,9 Psia) und 0,76 bar (11 Psia) durchgeführt wird, um eine konzentrierte L-Lysinbrühe zur Verfügung zu stellen, die zwischen 30 Gew.-% und 70 Gew.-% Feststoffe aufweist.

7. Das Verfahren von einem der Ansprüche 1 bis 6, worin der wesentliche Trocknungsschritt (c) aus Trommeltrocknen, Sprühtrocknen, Rotationstrocknen, Blechtrocknen, Tunneltrocknen und Sprühgranulation ausgewählt ist.

8. Das Verfahren gemäß Anspruch 7, worin der wesentliche Trocknungsschritt (c) Sprühgranulation ist.

9. Das Verfahren gemäß Anspruch 7, worin die Sprühgranulation die Schritte des Sprühens und Atomisierens der angereicherten L-Lysin-haltigen Fermentationsbrühe auf ein Fließbett von L-Lysinpartikeln umfasst, welche aus sprühgetrockneter angereicherter L-Lysin-haltiger Fermentationsbrühe gebildet wurden.

10. Das Verfahren gemäß Anspruch 9, worin die L-Lysinpartikel in einem Größenbereich zwischen ungefähr 100 Mikrometer und 177 Mikrometer liegen.

## Revendications

1. Procédé de production d'un complément alimentaire à base de L-lysine, comprenant :
(a) la détermination de la concentration en L-lysine, mesurée en pourcentage de base libre par kg de matière sèche, d'un bouillon de fermentation contenant de la L-lysine ;
(b) l'addition d'une L-lysine pure qui est choisie parmi le chlorhydrate de L-lysine et le sulfate de L-lysine, audit bouillon de fermentation contenant de la L-lysine, ladite L-lysine ajoutée étant en une quantité qui donne un complément alimentaire final à base de L-lysine ayant un titre en L-lysine dans une gamme comprise entre 35% et 80% de L-lysine, mesuré en pourcentage de base libre par kg de complément alimentaire final à base de L-lysine ; et
(c) le quasi-séchage du bouillon de L-lysine enrichi pour fournir le complément alimentaire final à base de L-lysine.

2. Procédé selon la revendication 1, dans lequel la L-lysine est ajoutée à un bouillon de fermentation concentré contenant de la L-lysine.

3. Procédé selon la revendication 1, dans lequel le quasi-séchage de l'étape (c) fournit le complément alimentaire final à base de L-lysine ayant un titre en L-lysine dans une gamme comprise entre 35% et 80% de L-lysine, mesuré en pourcentage de base libre par kg.

4. Procédé selon la revendication 2, dans lequel le bouillon de fermentation concentré est produit en évaporant un bouillon de fermentation contenant de la L-lysine afin d'éliminer l'eau.

5. Procédé selon la revendication 4, dans lequel l'évaporation est réalisée entre 60°C (140°F) et 101°C (214°F).

6. Procédé selon la revendication 4, dans lequel l'évaporation est réalisée dans une gamme de températures comprise entre 60°C (140°F) et 101°C (214°F) et sous un vide compris entre 0,2 bar (2,9 psia) et 0,76 bar (11 psia) pour fournir un bouillon de L-lysine concentré qui comprend entre 30% et 70% en poids de matières solides.

7. Procédé selon l'une quelconque des revendications 1 à 6, dans lequel le quasi-séchage de l'étape (c) est choisi parmi un séchage sur cylindres, un séchage par pulvérisation, un séchage par rotation, un séchage sur plateaux, un séchage en tunnel et une granulation par pulvérisation.

8. Procédé selon la revendication 7, dans lequel le quasi-séchage de l'étape (c) est une granulation par pulvérisation.

9. Procédé selon la revendication 7, dans lequel la granulation par pulvérisation comprend les étapes de pulvérisation et d'atomisation du bouillon de fermentation enrichi contenant de la L-lysine sur un lit fluidisé de particules de L-lysine qui ont été formées à partir d'un bouillon de fermentation contenant de la L-lysine, enrichi et séché par pulvérisation.

10. Procédé selon la revendication 9, dans lequel les particules de L-lysine sont dans une gamme de tailles comprise entre environ 100 microns et 177 microns.
